# EUROPEAN PATENT SPECIFICATION

(11) **EP 2 528 645 B1**
(45) Date of publication and mention of the grant of the patent: **20.11.2013**
(21) Application number: 11710272.3
(22) Date of filing: 27.01.2011
(51) Int. Cl.: A61M 25/00

(54) **CATHETER FOR THE PARENTERAL ADMINISTRATION OF BIOMEDICAL FLUIDS**
KATHETER ZUR PARENTERALEN VERABREICHUNG BIOMEDIZINISCHER FLÜSSIGKEITEN
CATHÉTER POUR ADMINISTRATION PARENTÉRALE DE FLUIDES BIOMÉDICAUX

(30) Priority: 28.01.2010 IT MO20100017
(43) Date of publication of application: 05.12.2012
(73) Proprietor: Evoluzione S.r.l., 00195 Roma (IT); De Lutio, Enrico, 05022 Amelia (TR) (IT); Pittiruti, Mauro, 00135 Roma (IT); Scoppettuolo, Giancarlo, 00157 Roma (IT); Brutti, Alberto, 60010 Ostra (AN) (IT)
(72) Inventor: PITTIRUTI, Mauro, I - 00135 Roma (IT); SCOPPETTUOLO, Giancarlo, I-00157 Roma (IT); BRUTTI, Barbara, I - 60030 San Marcello (AN) (IT)
(74) Representative: Brunacci, Marco
(86) International application number: PCT/IB2011/000129
(87) International publication number: WO 2011/092578

(56) References cited:
- EP-A1- 0 711 574
- WO-A1-94/28961
- WO-A1-2009/051969
- DE-A1- 10 243 208
- US-A1- 2007 016 124

## Description

### Technical Field

The present invention relates to a catheter for the parenteral administration of biomedical fluids.

### Background Art

With reference to the medical field, the need is known to use catheters for the parenteral administration of fluids to patients, that can be used in the medium term, in a continuous or discontinuous way, both in the intrahospital and extrahospital fields.

Known catheters generally comprise a flexible tube made of high biocompatibility materials and having, at one extremity, a connector that can be connected to a supply line of the fluid to be administered to the patient and, at an opposite extremity, a fluid outlet hole.

Such catheters are generally inserted by means of peripheral venous cannulation.

The need is also known to administer biomedical fluids to a patient by peripheral or central route depending on the type of therapy to be carried out and the type of biomedical fluid.

In particular, with reference to medium-term use catheters, central catheters are known, so-called PICC (Peripherally Inserted Central Catheters) and peripheral catheters, so-called Midline, usable according to the type of biomedical fluid to be administered.

More specifically, the use of central catheters envisages the insertion of the flexible tube in a peripheral vein of the patient and the positioning of the extremity having the fluid outlet hole near the joint between the superior *vena cava* and the right atrium of the heart.

The central catheters can be used, e.g., to administer chemotherapy drugs, inotropic drugs or, in general, to administer all those solutions and those drugs that are not compatible with peripheral venous route.

The use of peripheral catheters on the other hand envisages the insertion of the flexible tube in a peripheral vein of the patient's upper limb, until the extremity with fluid outlet hole is positioned near the axillary vein or the subclavian vein or, in any case, in a non-central position.

The peripheral catheters can be used e.g., for parenteral nutrition, for blood transfusions, for administering antibiotics or patient hydration fluids or, in general, for all those pharmacological and nutritional therapies compatible with peripheral venous route.

These catheters of known type do however have a number of drawbacks.

In particular, the need is known to administer different biomedical fluids to patients at the same time, both peripheral venous and central.

For patients in intensive care, e.g., it could be necessary to centrally administer inotropic drugs and, at the same time, antibiotics and blood by peripheral venous route.

A further example of such need is provided in the hemato-oncological field, in the case of having to centrally administer chemotherapy drugs and, at the same time, hemoderivatives or hydrating fluids by peripheral venous route.

This inevitably results in the use of at least two different catheters, a central catheter and a peripheral catheter, introduced inside distinct veins of the patient and both connected to the fluid supply line.

Consequently, in the case of the simultaneous peripheral venous and central administration of fluids, the operations of cannulation and connection to the supply line, already complicated in themselves, have to be performed for both the catheters.

The patient, furthermore, is considerably restricted in his/her movements due to the presence of two catheters which, what is more, are fitted at two distinct points of the same limb or, even, on two different limbs.

Another type of known catheters is described by EP 0711574, WO 2009/051969, US 2007/016124, WO 94/28961.

More in particular, these documents describe a relative catheter comprising two ducts separated the one from the other, having relative inlet and outlet mouths used in particular to carry out haemodialysis.

More in detail, the catheters described by these documents are connected to a haemodialysis machine and permit extracting the patient's blood through one of the two ducts and re-introducing the blood inside the same patient, following the treatment performed by the haemodialysis machine, through the other duct. These types of catheters are therefore generally used to treat blood.

These catheters too do however have a number of drawbacks.

Their conformation does in fact prevent them being used for the parenteral administration of biomedical fluids.

More in particular, this type of catheter does not allow the simultaneous peripheral venous and central administration of different biomedical fluids.

Furthermore, these catheters of known type are hard to insert inside the patient inasmuch as their outlet mouths are defined on relative planes substantially perpendicular to the longitudinal axes of the ducts themselves, and this produces a lower penetration capacity.

### Object of the Invention

The main aim of the present invention is to provide a catheter for the parenteral administration of biomedical fluids which allows effecting the simultaneous peripheral venous and central administration of biomedical fluids in an easier and more practical way with respect to the devices of known type.

Within this aim, an object of the present invention is to simplify operations concerning patient cannulation and connection to the supply line.

Another object of the present invention is to provide a catheter for the parenteral administration of biomedical fluids that allows limiting inconvenience for the patients in case of the simultaneous peripheral venous and central administration of biomedical fluids.

Another object of the present invention is to provide a catheter for the parenteral administration of biomedical fluids that allows overcoming the mentioned drawbacks of the state of the art within the ambit of a simple, rational, easy and effective to use as well as low cost solution.

The above objects are achieved by the present catheter, for the parenteral administration of biomedical fluids as further disclosed in claim 1, comprising at least a flexible tubular element with elongated shape having at least a first inlet mouth for a first biomedical fluid to be administered to a patient by central venous route, at least a first outlet mouth for said first fluid and at least a first connection duct between said first inlet mouth and said first outlet mouth, at least a second inlet mouth of a second biomedical fluid to be administered to a patient by peripheral venous route, at least a second outlet mouth of said second fluid and at least a second connection duct between said second inlet mouth and said second outlet mouth, characterised by the fact that the distance between said first outlet mouth and said second outlet mouth is greater or equal to 20 cm for the simultaneous positioning of said first outlet mouth inside a central vein of the patient and of said second outlet mouth inside a peripheral vein of the patient.

### Brief Description of the Drawings

Other characteristics and advantages of the present invention will become more evident from the description of a preferred, but not sole, embodiment of a catheter for the parenteral administration of biomedical fluids, illustrated purely as an example but not limited to the annexed drawings in which:
figure 1 is a side and partial section view of the catheter according to the invention;
figure 2 is a section view of the catheter according to the invention along the section plane II-II of figure 1;
figure 3 is a section view of the catheter according to the invention along the section plane III-III of figure 1.

### Embodiments of the Invention

With particular reference to such figures, globally indicated by 1 is a catheter usable for the simultaneous administration of biomedical fluids to a patient by central route and peripheral route.

The catheter 1 comprises a flexible tubular element 2 of elongated shape that can be inserted, at least in part, into a peripheral vein of the patient.

Usefully, the tubular element 2 can be completely made using biocompatible materials such as polyurethane or alternative materials, in such a way as to resist high pressures (up to 300 psi) and allow the administration of large fluid flows (up to 5 ml/sec).

The tubular element 2 has a first inlet mouth 3 for a first biomedical fluid which has to be administered to the patient by central venous route, a first outlet mouth 4 for the first fluid and a first duct 5 which longitudinally crosses the tubular element 2 and which is suitable for connecting the first inlet mouth 3 and the first outlet mouth 4.

To allow the simultaneous administration of a second biomedical fluid to the patient by peripheral venous route, the tubular element 2 also has a second inlet mouth 6 for the second fluid, a second outlet mouth 7 for the second fluid and a second duct 8 that longitudinally crosses part of the tubular element 2 and which is suitable for connecting the second inlet mouth 6 and the second outlet mouth 7.

According to the invention, the distance between the first outlet mouth 4 and the second outlet mouth 7 is greater or equal to 20 cm so as to allow the positioning of the first outlet mouth 4 in correspondence to a central vein of the patient and, at the same time, the positioning of the second outlet mouth 7 in correspondence to a peripheral vein of the patient.

Such positioning is done by fitting the tubular element 2 at least in part in a vein of the patient, e.g., by cannulation of a vein of an upper limb of the patient. Preferably, the distance between the first outlet mouth 4 and the second outlet mouth 7 is between 25 cm and 30 cm.

As described above, the tubular element 2 has an elongated shape and, consequently, it has a longitudinal axis. The distances expressed in the present description should be taken as axial distances, i.e., measured along the longitudinal axis of the tubular element 2.

The first and the second ducts 5 and 8, defined in the tubular element 2, also have relevant longitudinal axes.

This allows administering, at the same time, the first fluid by central venous route and the second fluid by peripheral venous route using only the catheter 1, with minimum inconvenience for the patient.

In particular, with not exclusive reference to the embodiment of the catheter 1 shown in the illustrations, the tubular element comprises:
- a first extremity 9, with a substantially pointed shape and having a first outlet mouth 4;
- a second extremity 10, opposite the first extremity 9 and having the first inlet mouth 3 and the second inlet mouth 6;
- a section 11, between the first and the second extremities 9 and 10, having the second outlet mouth 7.

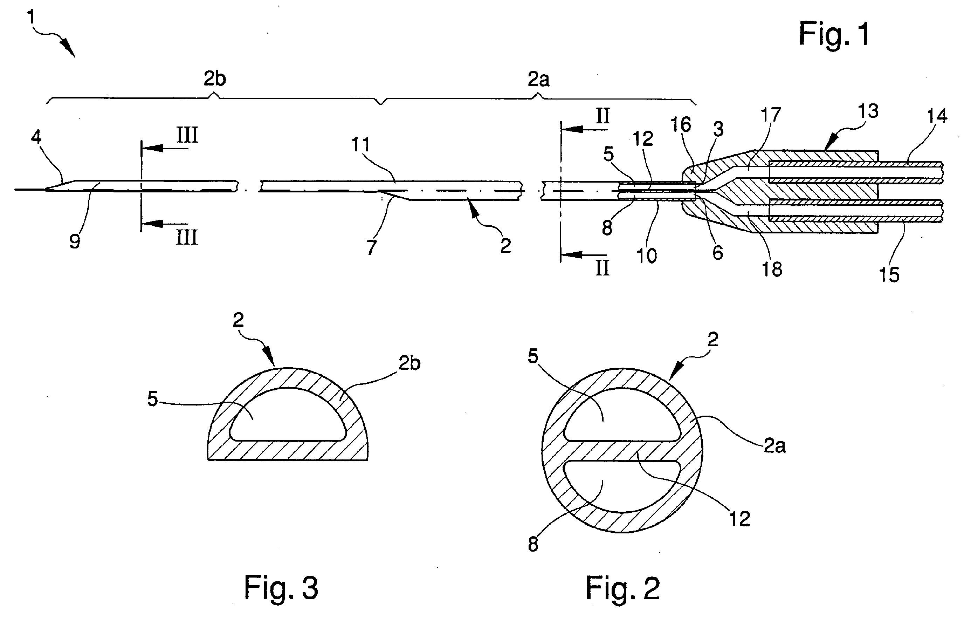

As shown in the figure 1, the second duct 8 connects the second inlet mouth 6 to the second outlet mouth 7 and extends inside a first portion 2a of the tubular element 2 which stretches from the second extremity 10 to the section 11.

The second duct 8, the length of which substantially corresponds to that of the first portion 2a, has a length substantially shorter or equal to 20 cm, preferably equal to 20 cm.

The first duct 5 connects the first inlet mouth 3 to the first outlet mouth 4 and extends along the entire tubular element 2, inside the above first portion 2a and inside a second portion 2b which stretches from the section 11 to the first extremity 9.

The first duct 5, the length of which substantially corresponds to the length of the section made up of the first and the second portions 2a and 2b, has a length substantially greater or equal to 40 cm, preferably between 45 cm and 50 cm. This particular conformation of the tubular element described herein allows the contemporaneous insertion of the first and the second ducts 5 and 8 inside a superior *vena cava* and a peripheral vein respectively.

Usefully, with reference to the particular embodiment of the catheter 1 shown in the illustrations, the first portion 2a of the tubular element 2 has a circular cross section and, inside it, the first duct 5 and the second duct 8 both have a semi-circular profile and are separated from each other by an inner wall 12.

The second portion 2b of the tubular element 2 has a semi-circular cross section and, inside it, the first duct 5 also has a semi-circular profile (so-called "D shape" in jargon).

Usefully, the section 11, making up the terminal part of the first portion 2a of the tubular element 2 and which has the second outlet mouth 7, has a cross section decreasing towards the second portion 2b.

This way, we have a gradual increase of the cross section from the first to the second portion 2a and 2b of the tubular element 2, consequently making easier the insertion and the sliding of the tubular element itself inside the patient's veins.

Advantageously, in the preferred embodiment shown in the illustrations, the first and the second outlet mouths 4 and 7 define relevant planes sloped with respect to the longitudinal axes of the corresponding ducts 5 and 8.

More in particular, the planes defined by the first and the second outlet mouths 4 and 7 are sloped the one with respect to the other by an angle bigger than 90°, so the first and the second outlet mouths themselves are opposing one another, i.e., turned in opposite directions with respect to a longitudinal median plane of the tubular element 2. This particular conformation of the outlet mouths 4 and 7 of the two ducts 5 and 8 permits easier penetration inside the relative veins and an efficient dispensing of the corresponding biomedical fluids.

It must be pointed out that the particular conformations of the first and the second portions 2a and 2b, of the first and the second ducts 5 and 8 and of the section 11 shown in the illustrations are such as to simplify and optimise the cannulation of the patient and the administration of the first and the second fluid. Different conformations cannot however be ruled out.

The catheter 1 furthermore comprises a connection element 13 suitable for connecting the second extremity 10 of the tubular element 2 to at least a supply device of the first and the second fluid to be administered to the patient.

In particular, the connection element 13 comprises a first inlet connector 14 and a second inlet connector 15 that can be connected to the above supply device and can be used to introduce the first biomedical fluid and the second biomedical fluid respectively, to be administered to the patient.

The connection element 13 also comprises an outlet connector 16 associated with the second extremity 10 of the tubular element 2 and a first and a second channel 17 and 18 suitable for connecting the first inlet connector 14 to the first inlet mouth 3 of the tubular element 2 and the second inlet connector 15 to the second inlet mouth 6 of the tubular element 2 respectively.

Advantageously, the inlet connectors 14 and 15 can have different letterings and/or colours for easier identification.

Preferably, the connection element 13 is made by overmoulding with the second extremity 10 of the tubular element 2. Different manufacturing methods cannot however be ruled out.

It has in fact been ascertained how the described invention achieves the proposed objects and in particular the fact is underlined that the invention allows the simultaneous peripheral venous and central venous administration of biomedical fluids while at the same time simplifying patient cannulation and supply line connection operations.

In fact, the use of just one catheter for the simultaneous peripheral venous and central venous administration of biomedical fluids considerably reduces inconvenience for patients during cannulation and allows the patients to move more freely during fluid administration.

## Claims

1. Catheter (1) for the parenteral administration of biomedical fluids, comprising at least a flexible tubular element (2) with elongated shape having at least a first inlet mouth (3) for a first biomedical fluid to be administered to a patient by central venous route, at least a first outlet mouth (4) for said first fluid and at least a first connection duct (5) between said first inlet mouth (3) and said first outlet mouth (4), at least a second inlet mouth (6) of a second biomedical fluid to be administered to a patient by peripheral venous route, at least a second outlet mouth (7) of said second fluid and at least a second connection duct (8) between said second inlet mouth (6) and said second outlet mouth (7), where the first and the second outlet mouths (4, 7) define relevant planes sloped with respect to the longitudinal axes of the corresponding ducts (5, 8), **characterised by** the fact that the distance between said first outlet mouth (4) and said second outlet mouth (7) is greater or equal to 20 cm for the simultaneous positioning of said first outlet mouth (4) inside a central vein of the patient and of said second outlet mouth (7) inside a peripheral vein of the patient and by the fact that the planes defined by the first and the second outlet mouths (4, 7) are sloped the one with respect to the other by an angle bigger than 90°, said first and second outlet mouths (4, 7) being opposite one another.

2. Catheter (1) according to claim 1, **characterised by** the fact that the distance between said first outlet mouth (4) and said second outlet mouth (7) is between 25 cm and 30 cm.

3. Catheter (1) according to claim 1 or 2, **characterised by** the fact that said tubular element (2) comprises at least a first extremity (9) having said first outlet mouth (4).

4. Catheter (1) according to one or more of the previous claims, **characterised by** the fact that said tubular element (2) comprises at least a second extremity (10) having at least one between said first and said second inlet mouth (3, 6).

5. Catheter (1) according to one or more of the previous claims, **characterised by** the fact that said tubular element (2) comprises at least a section (11) between said first and second extremities (9, 10) having said second outlet mouth (7).

6. Catheter (1) according to one or more of the previous claims, **characterised by** the fact that said second extremity (10) has said first inlet mouth (3) and said second inlet mouth (6), said second duct (8) extends inside a first portion (2a) of said tubular element (2), between said second extremity (10) and said section (11), and said first duct (5) extends inside said first portion (2a) and inside a second portion (2b), between said section (11) and said first extremity (9).

7. Catheter (1) according to one or more of the previous claims, **characterised by** the fact that said first portion (2a) has a substantially circular cross section.

8. Catheter (1) according to one or more of the previous claims, **characterised by** the fact that said first portion (2a) comprises at least an inner wall (12) to separate said first duct (5) from said second duct (8).

9. Catheter (1) according to one or more of the previous claims, **characterised by** the fact that said second portion (2b) has a substantially semi-circular cross section.

10. Catheter (1) according to one or more of the previous claims, **characterised by** the fact that at least one between said first duct (5) and said second duct (8) has a substantially semi-circular profile.

11. Catheter (1) according to one or more of the previous claims, **characterised by** the fact that said first extremity (9) has a substantially pointed shape.

12. Catheter (1) according to one or more of the previous claims, **characterised by** the fact that said section (11) having the second outlet mouth (7) represents the terminal part of said first portion (2a) and has a cross section substantially decreasing towards said second portion (2b).

13. Catheter (1) according to one or more of the previous claims, **characterised by** the fact that said second extremity (10) is associable with at least a supply device of at least one between said first and said second fluid.

14. Catheter (1) according to one or more of the previous claims, **characterised by** the fact that it comprises at least a connection element (13) for connecting said second extremity (10) of the tubular element (2) to at least a supply device of at least one between said first and said second fluid.

15. Catheter (1) according to one or more of the previous claims, **characterised by** the fact that said connection element (13) comprises at least a first inlet connector (14) of said first fluid and at least a second inlet connector (15) of said second fluid associable with said supply device, at least an outlet connector (16) associated with said second extremity (10) of the tubular element (2) and at least a first channel (17) and a second channel (18) suitable for connecting said first inlet connector (14) to said first inlet mouth (3) and said second inlet connector (15) to said second inlet mouth (6) respectively.

## Patentansprüche

1. Katheter (1) zur parenteralen Verabreichung biomedizinischer Fluide, mit wenigstens einem flexiblen Rohrelement (2) mit länglicher Form, das aufweist wenigstens eine erste Einlassöffnung (3) für ein erstes biomedizinisches Fluid, das einem Patienten über einen zentralvenösen Zugang verabreicht werden soll, wenigstens eine erste Auslassöffnung (4) für das erste Fluid und wenigstens eine erste Verbindungsleitung (5) zwischen der ersten Einlassöffnung (3) und der ersten Auslassöffnung (4), wenigstens eine zweite Einlassöffnung (6) für ein zweites biomedizinisches Fluid, das einem Patienten über einen periphervenösen Zugang verabreicht werden soll, wenigstens eine zweite Auslassöffnung (7) des zweiten Fluids und wenigstens eine zweite Verbindungsplatte (8) zwischen der zweiten Einlassöffnung (6) und der zweiten Auslassöffnung (7), wobei die erste und die zweite Auslassöffnung (4, 7) relevante Ebenen bilden, die in Bezug zu den Längsachsen der korrespondierenden Leitungen (5, 8) schräg sind, **gekennzeichnet durch** die Tatsache, dass der Abstand zwischen der ersten Auslassöffnung (4) und der zweiten Auslassöffnung (7) für das simultane Positionieren der ersten Auslassöffnung (4) innerhalb einer zentralen Vene des Patienten und der zweiten Auslassöffnung (7) innerhalb einer peripheren Vene des Patienten größer oder gleich 20 cm ist, und **durch** die Tatsache, dass die **durch** die erste und die zweite Auslassöffnung (4, 7) gebildeten Ebenen zueinander in einem Winkel größer als 90° geneigt sind, wobei die erste und zweite Auslassöffnung (4, 7) einander entgegengesetzt sind.

2. Katheter (1) nach Anspruch 1, **gekennzeichnet durch** die Tatsache, dass der Abstand zwischen der ersten Auslassöffnung (4) und der zweiten Auslassöffnung (7) zwischen 25 cm und 30 cm beträgt.

3. Katheter (1) nach Anspruch 1 oder 2, **gekennzeichnet durch** die Tatsache, dass das Rohrelement (2) wenigstens ein erstes Ende (9) mit der ersten Auslassöffnung (4) umfasst.

4. Katheter (1) nach ein oder mehreren der vorherigen Ansprüche, **gekennzeichnet durch** die Tatsache, dass das Rohrelement (2) wenigstens ein zweites Ende (10) umfasst, dass wenigstens eine der ersten und zweiten Einlassöffnung (3, 6) aufweist.

5. Katheter (1) nach ein oder mehreren der vorherigen Ansprüche, **gekennzeichnet durch** die Tatsache, dass das Rohrelement (2) wenigstens einen Abschnitt (11) zwischen dem ersten und zweiten Ende (9, 10) mit der zweiten Auslassöffnung (7) umfasst.

6. Katheter (1) nach ein oder mehreren der vorherigen Ansprüche, **gekennzeichnet durch** die Tatsache, dass das zweite Ende (10) die erste Einlassöffnung (3) und die zweite Einlassöffnung (6) aufweist, wobei sich die zweite Leitung (8) innerhalb eines ersten Bereichs (2a) des Rohrelements (2) zwischen dem zweiten Ende (10) und dem Abschnitt (11) erstreckt und sich die erste Leitung (5) innerhalb des ersten Bereichs (2a) und innerhalb eines zweiten Bereichs (2b) zwischen dem Abschnitt (11) und dem ersten Ende (9) erstreckt.

7. Katheter (1) nach ein oder mehreren der vorherigen Ansprüche, **gekennzeichnet durch** die Tatsache, dass der erste Bereich (2a) einen im Wesentlichen kreisförmigen Querschnitt hat.

8. Katheter (1) nach ein oder mehreren der vorherigen Ansprüche, **gekennzeichnet durch** die Tatsache, dass der erste Bereich (2a) wenigstens eine Innenwand (12) umfasst, um die erste Leitung (5) von der zweiten Leitung (8) zu trennen.

9. Katheter (1) nach ein oder mehreren der vorherigen Ansprüche, **gekennzeichnet durch** die Tatsache, dass der zweite Bereich (2b) einen im Wesentlichen halbkreisförmigen Querschnitt hat.

10. Katheter (1) nach ein oder mehreren der vorherigen Ansprüche, **gekennzeichnet durch** die Tatsache, dass wenigstens eine der ersten Leitung (5) und der zweiten Leitung (8) ein im Wesentlichen halbkreisförmiges Profil hat.

11. Katheter (1) nach ein oder mehreren der vorherigen Ansprüche, **gekennzeichnet durch** die Tatsache, dass das erste Ende (9) eine im Wesentlichen spitze Form hat.

12. Katheter (1) nach ein oder mehreren der vorherigen Ansprüche, **gekennzeichnet durch** die Tatsache, dass der Abschnitt (11) mit der zweiten Auslassöffnung (7) den Endteil des ersten Bereichs (2a) darstellt und einen Querschnitt hat, der in Richtung des zweiten Bereichs (2b) im Wesentlichen abnimmt.

13. Katheter (1) nach ein oder mehreren der vorherigen Ansprüche, **gekennzeichnet durch** die Tatsache, dass das zweite Ende (10) mit wenigstens einer Versorgungseinrichtung für wenigstens eines des ersten und zweiten Fluids verbunden werden kann.

14. Katheter (1) nach ein oder mehreren der vorherigen Ansprüche, **gekennzeichnet durch** die Tatsache, dass dieser wenigstens ein Verbindungselement (13) zum Verbinden des zweiten Endes (10) des Rohrelements (2) mit wenigstens einer Versorgungseinrichtung für wenigstens eines des ersten und zweiten Fluids umfasst.

15. Katheter (1) nach ein oder mehreren der vorherigen Ansprüche, **gekennzeichnet durch** die Tatsache, dass das Verbindungselement (13) aufweist wenigstens einen ersten Einlassverbinder (14) für das erste Fluid und wenigstens einen zweiten Einlassverbinder (15) für das zweite Fluid, die mit der Versorgungseinrichtung verbunden werden können, wenigstens einen Auslassverbinder (16), der mit dem zweiten Ende (10) des Rohrelements (2) verbunden werden kann und wenigstens einen ersten Kanal (17) und einen zweiten Kanal (18), die zum Verbinden des ersten Einlassverbinders (14) mit der ersten Einlassöffnung (3) bzw. des zweiten Einlassverbinders (15) mit der zweiten Einlassöffnung (6) geeignet sind.

## Revendications

1. Cathéter (1) pour l'administration parentérale de fluides biomédicaux, comprenant au moins un élément tubulaire souple (2) de forme allongée comportant au moins une première ouverture d'entrée (3) pour un premier fluide biomédical devant être administré à un patient par voie veineuse centrale, au moins une première ouverture de sortie (4) pour ledit premier fluide et au moins un premier conduit de liaison (5) entre ladite première ouverture d'entrée (3) et ladite première ouverture de sortie (4), au moins une deuxième ouverture d'entrée (6) d'un deuxième fluide biomédical devant être administré à un patient par voie veineuse périphérique, au moins une deuxième ouverture de sortie (7) dudit deuxième fluide et au moins un deuxième conduit de liaison (8) entre ladite deuxième ouverture d'entrée (6) et ladite deuxième ouverture de sortie (7), les première et deuxième ouvertures de sortie (4, 7) définissant des plans appropriés inclinés par rapport aux axes longitudinaux des conduits (5, 8) correspondants, **caractérisé par le fait que** la distance entre ladite première ouverture de sortie (4) et ladite deuxième ouverture de sortie (7) est supérieure ou égale à 20 cm pour le positionnement simultané de ladite première ouverture de sortie (4) à l'intérieur d'une veine centrale du patient et de ladite deuxième ouverture de sortie (7) à l'intérieur d'une veine périphérique du patient, et **par le fait que** les plans définis par les première et deuxième ouvertures de sortie (4, 7) sont inclinés l'un par rapport à l'autre selon un angle supérieur à 90°, lesdites première et deuxième ouvertures de sortie (4, 7) étant opposées l'une à l'autre.

2. Cathéter (1) selon la revendication 1, **caractérisé par le fait que** la distance entre ladite première ouverture de sortie (4) et ladite deuxième ouverture de sortie (7) est comprise entre 25 cm et 30 cm.

3. Cathéter (1) selon la revendication 1 ou 2, **caractérisé par le fait que** ledit élément tubulaire (2) comprend au moins une première extrémité (9) comportant ladite première ouverture de sortie (4).

4. Cathéter (1) selon une ou plusieurs des revendications précédentes, **caractérisé par le fait que** ledit élément tubulaire (2) comprend au moins une deuxième extrémité (10) comportant au moins l'une parmi ladite première et ladite deuxième ouverture d'entrée (3, 6).

5. Cathéter (1) selon une ou plusieurs des revendications précédentes, **caractérisé par le fait que** ledit élément tubulaire (2) comprend au moins une section (11) entre lesdites première et deuxième extrémités (9, 10) comportant ladite deuxième ouverture de sortie (7).

6. Cathéter (1) selon une ou plusieurs des revendications précédentes, **caractérisé par le fait que** ladite deuxième extrémité (10) comporte ladite première ouverture d'entrée (3) et ladite deuxième ouverture d'entrée (6), que ledit deuxième conduit (8) s'étend à l'intérieur d'une première partie (2a) dudit élément tubulaire (2), entre ladite deuxième extrémité (10) et ladite section (11), et que ledit premier conduit (5) s'étend à l'intérieur de ladite première partie (2a) et à l'intérieur d'une deuxième partie (2b), entre ladite section (11) et ladite première extrémité (9).

7. Cathéter (1) selon une ou plusieurs des revendications précédentes, **caractérisé par le fait que** ladite première partie (2a) a une section transversale sensiblement circulaire.

8. Cathéter (1) selon une ou plusieurs des revendications précédentes, **caractérisé par le fait que** ladite première partie (2a) comprend au moins une paroi intérieure (12) pour séparer ledit premier conduit (5) dudit deuxième conduit (8).

9. Cathéter (1) selon une ou plusieurs des revendications précédentes, **caractérisé par le fait que** ladite deuxième partie (2b) a une section transversale sensiblement semi-circulaire.

10. Cathéter (1) selon une ou plusieurs des revendications précédentes, **caractérisé par le fait qu'**au moins l'un parmi ledit premier conduit (5) et ledit deuxième conduit (8) a un profil sensiblement semi-circulaire.

11. Cathéter (1) selon une ou plusieurs des revendications précédentes, **caractérisé par le fait que** ladite première extrémité (9) a une forme essentiellement pointue.

12. Cathéter (1) selon une ou plusieurs des revendications précédentes, **caractérisé par le fait que** ladite section (11) comportant la deuxième ouverture de sortie (7) représente la partie terminale de ladite première partie (2a) et a une section transversale sensiblement décroissante en direction de ladite deuxième partie (2b).

13. Cathéter (1) selon une ou plusieurs des revendications précédentes, **caractérisé par le fait que** ladite deuxième extrémité (10) peut être associée à au moins un dispositif d'alimentation d'au moins l'un parmi ledit premier et ledit deuxième fluide.

14. Cathéter (1) selon une ou plusieurs des revendications précédentes, **caractérisé par le fait qu'**il comprend au moins un élément de connexion (13) destiné à relier ladite deuxième extrémité (10) de l'élément tubulaire (2) à au moins un dispositif d'alimentation d'au moins l'un parmi ledit premier et ledit deuxième fluide.

15. Cathéter (1) selon une ou plusieurs des revendications précédentes, **caractérisé par le fait que** ledit élément de connexion (13) comprend au moins un premier connecteur d'entrée (14) dudit premier fluide et au moins un deuxième connecteur d'entrée (15) dudit deuxième fluide, pouvant être associés audit dispositif d'alimentation, au moins un connecteur de sortie (16) associé à ladite deuxième extrémité (10) de l'élément tubulaire (2), et au moins un premier canal (17) et un deuxième canal (18) appropriés pour relier respectivement ledit premier connecteur d'entrée (14) à ladite première ouverture d'entrée (3) et ledit deuxième connecteur d'entrée (15) à ladite deuxième ouverture d'entrée (6).
